# EUROPEAN PATENT SPECIFICATION

(11) **EP 2 488 246 B1**
(45) Date of publication and mention of the grant of the patent: **28.08.2019**
(21) Application number: 09756810.9
(22) Date of filing: 16.10.2009
(51) Int. Cl.: A61M 35/00, B65D 83/14, G01F 11/02

(54) **DEVICE FOR DISPENSING ONE OR MORE SKIN TREATMENT SUBSTANCES**
VORRICHTUNG ZUR ABGABE VON EINER ODER MEHRERER HAUTBEHANDLUNGSSUBSTANZEN
DISPOSITIF POUR DISTRIBUER UNE OU PLUSIEURS SUBSTANCES DE TRAITEMENT DE LA PEAU

(43) Date of publication of application: 22.08.2012
(73) Proprietor: Sixtem Life S.r.l., 59100 Prato (PO) (IT)
(72) Inventor: OTTANELLI, Luciano, I-50019 Sesto Fiorentino (FI) (IT)
(74) Representative: Vinci, Marcello
(86) International application number: PCT/IB2009/054565
(87) International publication number: WO 2011/045630

(56) References cited:
- EP-A1- 0 307 127
- EP-A1- 0 367 604
- EP-A2- 0 260 067
- WO-A1-02/076854
- GB-A- 1 524 293
- GB-A- 2 087 355
- US-A- 6 113 008
- US-A1- 2004 139 965
- US-A1- 2008 154 210

## Description

### Field of application of the invention

The present invention can be generally applied in the sector of surface topic treatments of skin pathologies and diseases and, in particular, it concerns a device for dispensing one or more substances for skin treatments.

### State of the art

There are several examples of embodiment of devices for dispensing liquid and gaseous substances onto the skin, said substances being suitable for treating skin pathologies and diseases.

In particular, patent no. US5516505 describes a device for skin treatments comprising an elongated duct suited to convey a treatment fluid from the outlet valve of a pressurized container towards an applicator. The latter is made of a synthetic plastic foam. Patent EP0281212 describes an example of embodiment similar to the previous one, in which the fluid flows into a projection made of cotton wool, intended to come into contact with the skin surface to be treated.

An evident drawback that is common to both these solutions lies in that the treatment fluid is dispensed onto the skin through direct contact between the skin and the applicator. According to this application method, the quantity of fluid that is applied to the skin and the skin area treated depend on several factors that are rather difficult to control, for example the contact pressure between the skin and the applicator and the deformability of the latter. As a consequence of the above, the treated area is often larger than the area affected by the disease or pathology, and the treatment fluid is spread on an area that does not require any treatment. Furthermore, the quantity of fluid conveyed may not be sufficient for the skin area actually affected by the disease, which goes to the detriment of the effectiveness of the treatment.

A further drawback that is common to the two solutions mentioned above lies in that during application the treatment fluid may easily drip on the skin, thus staining the clothes in proximity to the treatment area.

Patent no. GB1163573 describes a device suitable for the treatment of delimited skin areas. The device is provided with a tubular sleeve having an open longitudinal end and the other end connected to the outlet valve of a pressurized container. The edge of the open longitudinal end is intended to rest on the skin at the level of the area to be treated, in such a way as to confine the fluid only to the skin area delimited by the edge and to prevent the fluid from coming into contact with other areas of the skin.

A clear drawback posed by this last solution is represented by the fact that the quantity of treatment fluid that reaches the skin area delimited by the edge is not controlled and may be excessive or insufficient, depending on how long the outlet valve of the pressurized container remains open. In particular, if the quantity of fluid let out is excessive, once the edge of the open longitudinal end has been moved away from the skin after application, the excess fluid may spread in the surrounding areas, thus making it useless to confine the fluid with the edge of the open longitudinal end and making application potentially dangerous also in this case for the clothes near the area to be treated.

According to the state of the art, furthermore, there are many embodiments of devices for applying skin treatment fluids by spraying. In these solutions the fluid is atomized after coming out of a valve of a pressurized container. The atomized fluid is directed toward the skin in proximity to the area to be treated.

A drawback posed by this solution lies in that the application area is generally much larger than necessary and cannot be easily and precisely circumscribed. A further drawback is represented by the fact that, also in this case, the quantity of fluid dispensed depends on how long the user keeps the valve open, thus considerably increasing the risk of making the treatment ineffective.

See further EP0307127 A1 and GB2087355 A.

### Description of the invention

A primary object of the invention is to eliminate the drawbacks mentioned above by means of a device for dispensing one or more skin treatment substances that is easy to use and at the same time highly effective against localized skin pathologies and diseases.

A particular object of the invention is to carry out a device capable of concentrating the treatment fluid only on the skin areas that actually need it.

A further object of the invention is to propose a device that can dispense a predefined quantity of treatment substance onto the skin, thus guaranteeing easy and safe application and avoiding useless wastes.

Another specific object of the invention is to carry out a device capable of drastically reducing the risk of staining clothes in proximity to the skin area to be treated.

These objects, as well as others that will be highlighted in greater detail below, are achieved by a device, as claimed in claim 1, for dispensing one or more skin treatment substances, comprising a reservoir suited to contain the treatment substances, an element for dispensing the substances onto the skin, a valve suited to properly connect said container to said dispensing element, and characterized in that said valve comprises a dosage chamber having a predetermined volume suited to contain one portion of the treatment substance, and substance passage means suited to alternately place said dosage chamber in fluid communication with said container in order to fill it or with said dispensing element in order to dispense the treatment substances.

Thanks to this particular configuration it will be possible to properly dispense the correct portion of treatment substance onto the skin and to maximize the effectiveness of the treatment.

The device described above is used in combination with an innovative formula, focusing in particular on the treatment of rhagades and small injuries, and extending use to the cure and treatment of other skin diseases.

Products are known which are used in relation to rhagades and are described here below in order to better explain the innovative idea proposed by the invention compared to pre-existing products available on the market.

There are hydrocolloid plasters shaped like a half moon which are suited to cover rhagades on the fingers of the hand and the nail contour. Said plasters are difficult to apply and they don't stick for long.

Plasters and treatments in liquid solution are also known, which are applied in a traditional way by means of a brush or spatula that is used repeatedly to apply all the doses contained in the package. The above mentioned spatula is internally connected to the container closing cap, and after use it is stored inside said cap and remains in contact with the product.

Spray plasters are also known, which are applied through a continuous valve and allow application in undetermined quantities.

The invention described herein, in spray solution for the treatment of rhagades and small injuries, comprises a combination of said device and a medicament, with advanced formulation in a spray solution, practical and effective, suited to be transformed into a protective film against germs and bacteria, which thanks to its special formula favours cicatrization and prevents rhagades-injuries from reappearing. The device with predetermined dosage makes it possible to use the product in the correct pre-established quantity, that is, the quantity that is necessary and sufficient to treat rhagades and small injuries, avoiding useless product wastes.

The device is structured in such a way as to be activated with overturned bottle, to facilitate dispensing and avoid spraying the product into the eyes, thus ensuring safer and more appropriate use.

The new dispensing system allows the product to be applied through the air, with a direct liquid jet, avoiding the use of further accessories (brush-spatula, etc.). Therefore, the device also ensures more hygiene during use and therefore can also be used interpersonally (for example, within the same family).

The device is provided with a suitable cap, to be used to cover the applicator nozzle, in order to hygienically protect the device after use.

The advanced formulation medicament contained in the bottle is suitable for treating rhagades and small injuries and contains eugenol and panthenol.

Eugenol (C₁₀H₁₀O₂) is an hydroxilated aromatic compound belonging to the chemical class of allylbenzenes. It is an oily liquid, almost transparent or light yellow in colour, which is extracted from some essential oils, in particular from clove oil and cinnamon. It is hardly soluble in water and soluble in organic solvents, has a pleasant colour and is spicy, similar to clove. Eugenol also has disinfecting properties and light local anaesthetic and anti-inflammatory properties.

Panthenol is an active principle widely used in cosmesis for its hydrating and soothing action. Technically speaking, it is provitamin B5, the substance that must be transformed into vitamin by the organism before being used, and has good anti-inflammatory properties; it serves its function by intensifying lipid synthesis and promoting proliferation of fibroblasts.

### Brief description of the drawings

The characteristics of the new dispensing device will be highlighted in greater detail in the following description, with reference to the drawings attached as non-limiting examples.
Figure 1 shows an overall exploded view of the device;
Figure 2 shows a section view of the valve in retracted position;
Figure 3 shows a section view of the valve in extended position;
Figure 4 shows a section view of the dispensing element with side dispensing duct.

### Detailed description of a preferred embodiment of the invention

The device is described with particular reference to the enclosed figures, the reference numbers used in the description and in the claims are used to improve the intelligibility of the invention and do not constitute a limitation to the claimed scope of protection.

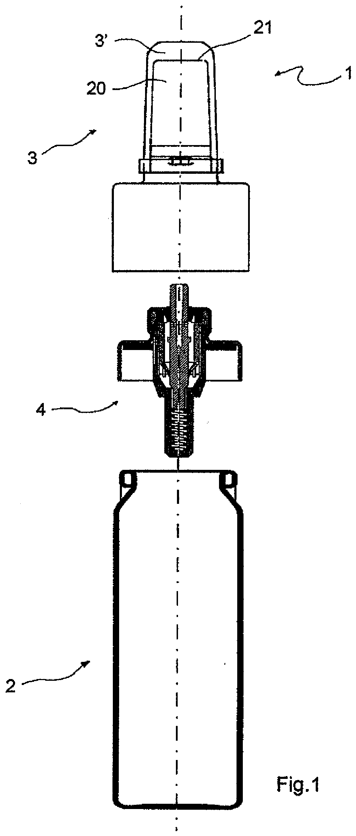

The device carried out according to the invention, as claimed in claim 1, for dispensing one or more skin treatment substances is indicated as a whole by reference number 1. The treatment substances can be gaseous or liquid, or a mixture of gaseous and/or liquid and/or solid components. The device 1 comprises a reservoir 2 suited to contain the treatment substances, a dispensing element 3 suited to apply the substances to the skin, and a valve 4 capable of properly connecting the reservoir 2 to the dispensing element 3 (Figure 1). A cap 3' can also be provided to cover and protect the dispensing element 3.

A characteristic of the invention lies in that the valve 4 comprises a dosage chamber 5 having a predetermined volume, such that it contains an optimal quantity of the treatment substances. The valve 4 also comprises passage means 6 suited to allow the tratment substances to pass from the reservoir 2 to the dosage chamber 5 while the latter is being filled, or alternatively from the dosage chamber 5 to the dispensing element 3 while the treatment substances are dispensed onto the skin.

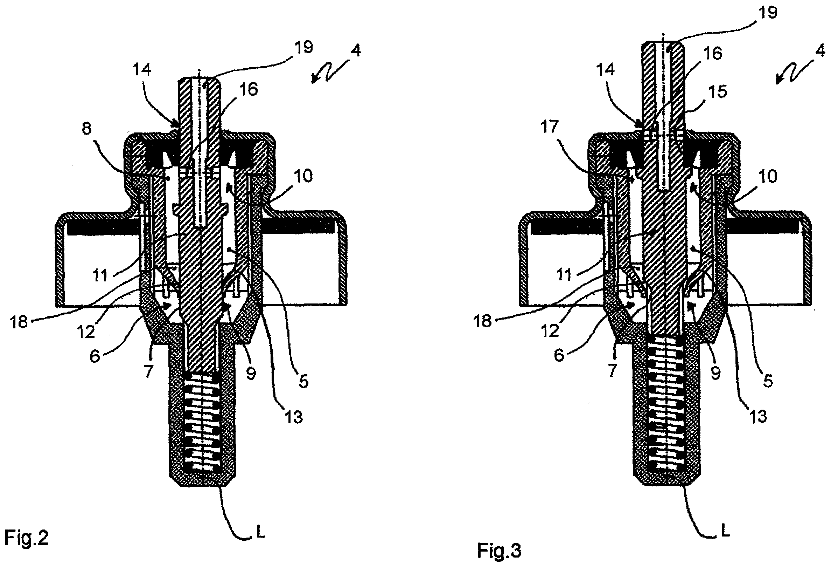

In greater detail, the passage means 6 comprises a filling path 7 (Figure 3) designed to be active during the filling of the dosage chamber 5 and an outlet path 8 (Figure 2) designed to empty the dosage chamber 5 while the substances are being dispensed. The filling path 7 and the outlet path 8 are never active at the same time, which prevents uncontrolled passage of the treatment substances directly from the reservoir 2 to the dispensing element 3.

This behaviour can be obtained through first closing means 9 capable of selectively obstructing the filling path 7 while the treatment substances are being dispensed, and through second closing means 10 capable of selectively obstructing the outlet path 8 while the dosage chamber 5 is being filled.

The valve 4 comprises a stem 11 constrained to the main body of the valve 4 so as to slide along a longitudinal axis L. The stem 11 moves between an extended position (Figure 3) during the filling of the dosage chamber 5 and a retracted position (Figure 2) during application of the treatment substances to the skin.

The first closing means 9 comprises an external wall 12 of the stem 11 and an elastic wall 13 of the dosage chamber 5, which come into contact when the stem 11 is in retracted position. Said contact can take place only during the dispensing stage and makes it possible to obstruct the filling path 7, thus preventing the substances present in the reservoir 2 from entering the dosage chamber 5 and flowing onto the skin in an uncontrolled manner.

In greater detail, the stem 11 slides inside a longitudinal hole 14 provided in the valve 4. The inner wall of the longitudinal hole 14 forms a sliding wall 15 for the stem 11. Furthermore, the second closing means 10 comprises at least one transverse channel 16 created in the stem 11. Said transverse channel 16 is positioned on the stem 11 in a longitudinal position that is such as to allow the channel to be obstructed by the sliding wall 15 when the stem 11 is in extended position. In this way, during the filling of the dosage chamber 5, the treatment substances cannot pass through the transverse channel 16 and cannot flow out through the dispensing element 3. On the contrary, when the stem 11 is in retracted position (Figure 2), the transverse channel 16 places the dosage chamber 5 in communication with the dispensing element, thus allowing the treatment substances to flow out.

As shown in Figure 3, the dosage chamber 5 has a longitudinal end portion 17 nearer to the dispensing element 3 and a portion 18 nearer to the reservoir, situated on the opposite side along the longitudinal axis L. A further special characteristic of the invention lies in that the transverse channel 16 is positioned on the stem 11 in such a way as to be at the level of the longitudinal end portion 17 of the dosage chamber 5 when the stem 11 is in retracted position. This features allows the device of the invention to be advantageously used with the dispensing element 3 directed downwards, ensuring substantially complete emptying of the dosage chamber 5.

The outlet path 8 comprises a longitudinal outlet duct 19, and the dispensing element 3 may comprise a longitudinal dispensing duct substantially aligned with the outlet duct 19. This detail favours the rapid and complete discharge of the substance from the dosage chamber 5 during the dispensing stage.

According to a preferred embodiment of the invention, the longitudinal dispensing duct of the dispensing element 3 may have an open longitudinal end 20 with a substantially plane edge 21 suited to be rested on the skin during the dispensing stage. This configuration makes it possible to confine the treatment substances inside the area defined by the edge 21. Furthermore, the combination of a precise dosage, obtained through the dosage chamber 5, with a defined application area makes it possible to apply an optimal quantity of substances, thus obtaining a correct thickness of the substance layer applied to the skin.

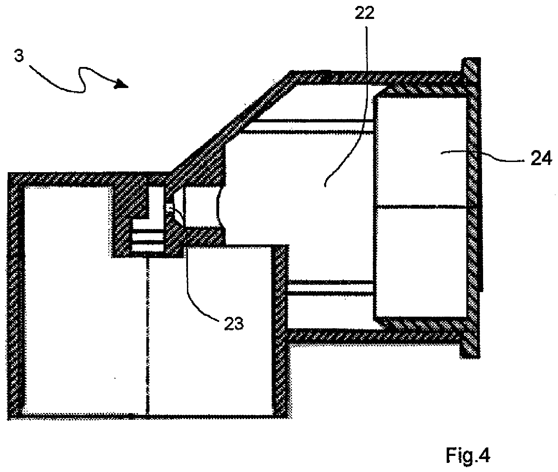

According to an alternative embodiment of the device of the invention, the dispensing element 3 may comprise a side dispensing duct 22 arranged crosswise with respect to the longitudinal axis L, in such a way as to ensure that the treatment substances can be comfortably applied to the skin even when the available space is limited (Figure 4). The dispensing element 3 may also comprise a substantially curved internal path 23 suited to place the longitudinal outlet duct 19 in communication with the side dispensing duct 22, thus allowing the passage of the treatment substances. Also in this embodiment a cap 24 can be provided, suited to selectively close the side dispensing duct 22.

A method for using the device of the invention is described here below. The method for dispensing one or more skin treatment substances comprises the following stages:
a) the edge 21 of the dispensing element 3 is placed into contact with the skin in such a way as to delimit the area to be treated;
b) to ensure optimal dispensing of mixtures of gaseous, liquid and solid substances, the longitudinal axis L can be substantially aligned with the direction orthogonal to the skin at the height of the surface to be treated, and with the local vertical direction. Furthermore, the dispensing element 3 can be directed downwards, in such a way as to guarantee the optimal emptying of the dosage chamber 5;
c) the dispensing element 3 can be pressed against the valve 4 in such a way as to make the stem 11 pass from the extended configuration to the retracted configuration to dispense the treatment substances.

The above clearly shows that the device carried out according to the invention achieves the set objects and in particular allows a pre-established quantity of treatment substances to be dispensed onto a delimited skin area, thus guaranteeing high effectiveness of the treatment.

Therefore, with reference to the preceding description, the following claims are expressed.

## Claims

1. Device for dispensing one or more skin treatment substances, comprising a formula for the treatment of rhagades and small injuries, and also for the cure and treatment of other skin diseases, said formula including eugenol and panthenol, said device also comprising a reservoir (2) for the treatment substances, a dispensing element (3) suited to dispense the substances onto the skin, a valve (4) suited to properly connect said reservoir (2) to said dispensing element (3), and wherein said valve (4) comprises:
- a dosage chamber (5) having a predetermined volume suited to contain one portion of the treatment substances,
- substance passage means (6) suited to alternatively place said dosage chamber (5) in fluid communication with said reservoir (2) in order to fill it or with said dispensing element (3) in order to dispense the treatment substances.
and wherein said passage means (6) comprise:
- a filling path (7) suited to fill said dosage chamber (5) and first closing means (9) suited to selectively obstruct said filling path (7) while the treatment substances are being dispensed;
- an outlet path (8) suited to empty said dosage chamber (5) while the substances are being dispensed and second closing means (10) suited to selectively obstruct said outlet path (8) while said dosage chamber (5) is being filled;
- a stem (11) slidingly constrained along a longitudinal axis (L) between an extended position for filling said dosage chamber (5) and a retracted position for dispensing said treatment substances, and wherein the sliding movement of said stem (11) takes place through contact and pressure of the end of said stem (11) onto the surface to be treated;
and wherein said valve (4) also comprises a longitudinal hole (14) defining a sliding wall (15) suited to slidingly house said stem (11), said second closing means (10) comprising at least one transverse channel (16) created in said stem (11) and having a longitudinal position suited to allow it to be selectively obstructed by said sliding wall (15) when said stem (11) is in said extended position, wherein said first closing means (9) comprise an external wall (12) of said stem (11) suited to come into selective contact with a corresponding elastic wall (13) of said dosage chamber (5) when said stem (11) is in said retracted position to obstruct said filling path (7),
said passage means (6) define a chamber having a constant and predetermined volume and wherein said dosage chamber (5) has a longitudinal end portion (17) in proximity to said dispensing element (3), said transverse channel (16) being positioned on said stem (11) in such a way as to be at the level of said longitudinal end portion (17) of said dosage chamber (5) when said stem (11) is in said retracted position, allowing for use with said dispensing element (3) directed downwards, ensuring complete emptying of the dosage chamber and wherein said reservoir (2) contains said formulation in spray solution for the treatment of rhagades and small injuries, suited to be transformed into a protective film against germs and bacteria, and suited to favour cicatrization and prevent rhagades-injuries from reappearing.

2. Device according to claim 1, **characterized in that** said valve (4) comprises a longitudinal outlet duct (19), said dispensing element (3) comprising a longitudinal dispensing duct substantially aligned with said outlet duct (19).

3. Device according to claim 2, **characterized in that** said dispensing duct has an open longitudinal end (20) with a substantially plane edge (21) suited to be rested on the skin during the dispensing operation.

4. Device according to one or more of the preceding claims, **characterized in that** said dispensing element (3) comprises a side dispensing duct (22) arranged crosswise with respect to said longitudinal axis L, and a substantially curved internal path (23) suited to place said longitudinal outlet duct (19) in communication with said side dispensing duct (22).

5. Device according to one or more of the preceding claims, suitable for dispensing one or more skin treatment substances, **characterized in that** it comprises:
- said edge (22) of said dispensing element (3), suited to be placed into contact with the skin in such a way as to delimit the area to be treated;
- said longitudinal axis (L), suited to be substantially aligned with the direction orthogonal to the skin at the level of the area to be treated and with the local vertical direction, said dispensing element (3) being directed downwards;
- said dispensing element (3), suited to be pressed against said valve (4) in such a way as to translate said stem (11) from said extended configuration, wherein said outlet path (8) is obstructed by the wall (15) of said hole (14), to said retracted configuration, wherein said outlet path (8) is in communication with said dosage chamber (5), in order to dispense the treatment substances.

## Patentansprüche

1. Vorrichtung zur Ausgabe einer oder mehrerer Substanzen für die Behandlung der Haut, eine Rezeptur für die Behandlung von Rhagaden und kleinen Verletzungen umfassend, und ebenso für die Heilung und Behandlung anderer Hautkrankheiten, wobei die besagte Rezeptur Eugenol und Panthenol umfasst, wobei die besagte Vorrichtung auch ein Reservoir (2) für die Behandlungssubstanzen umfasst, sowie ein Ausgabeelement (3), dazu geeignet, die Substanzen auf die Haut abzugeben, ein Ventil (4), dazu geeignet, das besagte Reservoir (2) korrekt mit dem besagten Ausgabeelement (3) zu verbinden, und wobei das besagte Ventil (4) Folgendes umfasst:
- eine Dosierkammer (5) mit einem vorbestimmten Volumen, dazu geeignet, eine Portion der Behandlungssubstanzen zu enthalten,
- Substanzdurchgangsmittel (6), dazu geeignet, die besagte Dosierkammer (5) alternativ mit dem besagten Reservoir (2), um es zu füllen, oder mit dem besagten Ausgabeelement (3), um die Behandlungssubstanzen auszugeben, in Fluid-Kommunikation zu versetzen,
und wobei die besagten Durchgangsmittel (6) Folgendes umfassen:
- einen Füllweg (7), dazu geeignet, die besagte Dosierkammer (5) zu füllen, und erste Verschlussmittel (9), dazu geeignet, wahlweise den besagten Füllweg (7) zu versperren, während die Behandlungssubstanzen ausgegeben werden;
- einen Auslassweg (8), dazu geeignet, die besagte Dosierkammer (5) zu leeren, während die Substanzen ausgegeben werden, und zweite Verschlussmittel (10), dazu geeignet, wahlweise den besagten Auslassweg (8) zu versperren, während die besagte Dosierkammer (5) gefüllt wird;
- einen Schaft (11), der entlang einer Längsachse (L) zwischen einer ausgezogenen Position zur Füllung der besagten Dosierkammer (5) und einer eingezogenen Position zur Ausgabe der besagten Behandlungssubstanzen gleitend befestigt ist, und wobei die Gleitbewegung des besagten Schafts (11) durch Kontakt und Druck des Endes des besagten Schafts (11) mit der/auf die zu behandelnde Oberfläche stattfindet;
und wobei das besagte Ventil (4) auch eine längliche Bohrung (14) umfasst, die eine gleitende Wand (15) definiert, dazu geeignet, den besagten Schaft (11) gleitend aufzunehmen, wobei die besagten zweiten Verschlussmittel (10) wenigstens einen in dem besagten Schaft (11) ausgeführten Querkanal (16) umfassen und eine Längsposition aufweisen, die geeignet ist, wahlweise seine Versperrung durch die besagte gleitende Wand (15) zu erlauben, wenn der besagte Schaft (11) sich in der besagten, ausgezogenen Position befindet, wobei die besagten ersten Verschlussmittel (9) eine Außenwand (12) des besagten Schafts (11) umfassen, dazu geeignet, wahlweise in Kontakt mit einer entsprechenden, elastischen Wand (13) der besagten Dosierkammer (5) zu kommen, wenn der besagte Schaft (11) sich in der besagten eingezogenen Position befindet, um den besagten Füllweg (7) zu versperren,
wobei die besagten Durchgangsmittel (6) eine Kammer definieren, die ein konstantes und vorbestimmtes Volumen aufweist
und wobei die besagte Dosierkammer (5) in der Nähe des besagten Ausgabeelements (3) einen länglichen Endabschnitt (17) aufweist, wobei der besagte Querkanal (16) derart an dem besagten Schaft (11) positioniert ist, dass er sich auf der Ebene des besagten länglichen Endabschnitts (17) der besagten Dosierkammer (5) befindet, wenn der besagte Schaft (11) sich in der besagten eingezogenen Position befindet, und den Gebrauch mit dem besagten Ausgabeelement (3) nach unten gerichtet erlaubt, unter Sicherstellung der vollständigen Leerung der Dosierkammer, und wobei das besagte Reservoir (2) die besagte Rezeptur in Spraylösung zur Behandlung von Rhagaden und kleinen Verletzungen enthält, dazu geeignet, in einen Schutzfilm gegen Keime und Bakterien verwandelt zu werden, und dazu geeignet, die Vernarbung zu fördern und das Wiederauftreten von Rhagaden/Verletzungen zu verhindern.

2. Vorrichtung nach Patentanspruch 1, **dadurch gekennzeichnet, dass** das besagte Ventil (4) eine längliche Auslassleitung (19) umfasst, wobei das besagte Ausgabeelement (3) eine längliche Ausgabeleitung umfasst, die im Wesentlichen auf die besagte Auslassleitung (19) ausgerichtet ist.

3. Vorrichtung nach Patentanspruch 2, **dadurch gekennzeichnet, dass** die besagte Ausgabeleitung ein offenes, längliches Ende (20) mit einer im Wesentlichen flachen Kante (21) aufweist, die dazu geeignet ist, während des Ausgabevorgangs auf der Haut aufgesetzt zu werden.

4. Vorrichtung nach einem oder mehreren der vorstehenden Patentansprüche, **dadurch gekennzeichnet, dass** das besagte Ausgabeelement (3) eine seitliche Ausgabeleitung (22) umfasst, die quer zu der besagten Längsachse L angeordnet ist, sowie einen im Wesentlichen gebogenen Innenweg (23), dazu geeignet, die besagte längliche Auslassleitung (19) mit der besagten seitlichen Ausgabeleitung (22) in Kommunikation zu versetzen.

5. Vorrichtung nach einem oder mehreren der vorstehenden Patentansprüche, dazu geeignet, eine oder mehrere Hautbehandlungssubstanzen auszugeben, **dadurch gekennzeichnet, dass** sie Folgendes umfasst:
- die besagte Kante (22) des besagten Ausgabeelements (3), dazu geeignet, derart mit der Haut in Kontakt versetzt zu werden, dass sie den zu behandelnden Bereich begrenzt;
- die besagte Längsachse (L), dazu geeignet, im Wesentlichen in der zu der Haut auf der Ebene des zu behandelnden Bereichs orthogonalen Richtung und in der örtlich senkrechten Richtung ausgerichtet zu werden, da das besagte Ausgabeelement (3) nach unten gerichtet ist;
- das besagte Ausgabeelement (3), dazu geeignet, derart gegen das besagte Ventil (4) gepresst zu werden, dass der besagte Schaft (11) aus der besagten ausgezogenen Konfiguration, in welcher der besagte Auslassweg (8) durch die Wand (15) der besagten Bohrung (14) versperrt ist, in die besagte eingezogene Konfiguration bewegt wird, wobei der besagte Auslassweg (8) in Kommunikation mit der besagten Dosierkammer (5) ist, um die Behandlungssubstanzen auszugeben.

## Revendications

1. Dispositif pour la distribution d'une ou plusieurs substances pour le traitement de la peau, comprenant une formulation pour le traitement de rhagades et de petites blessures, et en outre pour le soin et le traitement d'autres maladies de la peau, ladite formulation comprenant eugénol et panthénol, ledit dispositif comprenant également un réservoir (2) pour les substances de traitement, un élément de distribution (3) indiqué pour distribuer les substances sur la peau, une soupape (4) apte à relier adéquatement ledit réservoir (2) audit élément de distribution (3), et où ladite soupape (4) comprend :
- une chambre de dosage (5) ayant un volume prédéterminé pour contenir une dose des substances de traitement,
- des moyens de passage des substances (6) aptes à mettre alternativement ladite chambre de dosage (5) en communication fluide avec ledit réservoir (2) pour son remplissage ou avec ledit élément de distribution (3) pour la distribution des substances de traitement,
et où lesdits moyens de passage (6) comprennent :
- un parcours de remplissage (7) indiqué pour remplir ladite chambre de dosage (5) et des premiers moyens de fermeture (7) durant la distribution des substances de traitement ;
- un parcours de sortie (8) apte à vider ladite chambre de dosage (5) durant la distribution des substances et des deuxièmes moyens de fermeture (10) indiqués pour obstruer sélectivement ledit parcours de sortie (8) durant le remplissage de ladite chambre de dosage (5) ;
- une tige (11) bloquée de manière coulissante le long d'un axe longitudinal (L) entre une position étendue de remplissage de ladite chambre de dosage (5) et une position rentrée pour la distribution desdites substances de traitement, et où le mouvement de coulissement de ladite tige (11) se vérifie par contact et pression de l'extrémité de ladite tige (11) sur la surface à traiter ;
et où ladite soupape (4) comprend en outre un trou longitudinal (14) définissant une paroi de coulissement (15) apte à loger de manière coulissante ladite tige (11), lesdits deuxièmes moyens de fermeture (10) comprenant au moins un canal transversal (16) réalisé sur ladite tige (11) et ayant une position longitudinale apte à consentir son obstruction sélective par ladite paroi de coulissement (15) quand ladite tige (11) se trouve dans ladite position étendue,
où
lesdits premiers moyens de fermeture (9) comprennent une paroi extérieure (12) de ladite tige (11) indiquée pour entrer en contact sélectif avec une paroi élastique correspondante (13) de ladite chambre de dosage (5) quand ladite tige (11) se trouve dans ladite position rentrée pour obstruer ledit parcours de remplissage (7), lesdits moyens de passage (6) définissent une chambre ayant un volume constant et prédéterminé et où ladite chambre de dosage (5) présente une portion d'extrémité longitudinale (17) à proximité dudit élément de distribution (3), ledit canal transversal (16) étant positionné sur ladite tige (11) de manière à résulter à hauteur de ladite portion d'extrémité longitudinale (17) de ladite chambre de dosage (5) quand ladite tige (11) se trouve dans ladite position rentrée, en consentant l'usage avec ledit élément de distribution (3) tourné vers le bas, en garantissant le vidage complet de la chambre de dosage et où ledit réservoir (2) contient ladite formulation en solution de pulvérisation pour le traitement de rhagades et de petites blessures, indiquée pour être transformée en une pellicule de protection contre germes et bactéries, et indiquée pour favoriser la cicatrisation et empêcher que des rhagades et des blessures puissent resurgir.

2. Dispositif selon la revendication 1, **caractérisé en ce que** ladite soupape (4) comprend un conduit de sortie longitudinal (19), ledit élément de distribution (3) comprenant un conduit de distribution longitudinal essentiellement aligné avec ledit conduit de sortie (19).

3. Dispositif selon la revendication 2, **caractérisé en ce que** ledit conduit de distribution présente une extrémité longitudinale ouverte (20) avec un bord essentiellement plan (21) apte à être posé sur la peau durant l'opération de distribution.

4. Dispositif selon l'une ou plusieurs des revendications précédentes, **caractérisé en ce que** ledit élément de distribution (3) comprend un conduit de distribution latéral (22) disposé transversalement par rapport audit axe longitudinal L, et un parcours intérieur essentiellement courbe (23) apte à mettre ledit conduit de sortie longitudinal (19) en communication avec ledit conduit de distribution latéral (22).

5. Dispositif selon l'une ou plusieurs des revendications précédentes, apte à distribuer une ou plusieurs substances pour le traitement de la peau, **caractérisé en ce qu'**il comprend :
- ledit bord (22) dudit élément de distribution (3), indiqué pour être mis en contact avec la peau de manière à délimiter la zone à traiter ;
- ledit axe longitudinal (L), indiqué pour être essentiellement aligné avec la direction orthogonale à la peau à hauteur de la zone à traiter et avec la direction verticale locale, ledit élément de distribution (3) étant tourné vers le bas ;
- ledit élément de distribution (3), indiqué pour être appuyé contre ladite soupape (4) de manière à déplacer ladite tige (11) de ladite configuration étendue, où ledit parcours de sortie (8) est obstrué par la paroi (15) dudit trou (14), à ladite configuration rentrée, où ledit parcours de sortie (8) est en communication avec ladite chambre de dosage (5) de manière à distribuer les substances de traitement.
